(19) Europäisches Patentamt
European Patent Office
Office européen des brevets

(11) **EP 3 876 839 B1**

(12) **EUROPÄISCHE PATENTSCHRIFT**

(45) Veröffentlichungstag und Bekanntmachung des Hinweises auf die Patenterteilung:
**03.01.2024 Patentblatt 2024/01**

(21) Anmeldenummer: **19801871.5**

(22) Anmeldetag: **11.11.2019**

(51) Internationale Patentklassifikation (IPC):
**A61B 5/11** *(2006.01)* **A61B 5/00** *(2006.01)*

(52) Gemeinsame Patentklassifikation (CPC):
**A61B 5/1102;** A61B 5/6891; A61B 5/6892;
A61B 5/6894; A61B 2562/0247

(86) Internationale Anmeldenummer:
**PCT/EP2019/080908**

(87) Internationale Veröffentlichungsnummer:
**WO 2020/094888 (14.05.2020 Gazette 2020/20)**

(54) **SENSOROBERFLÄCHE**

SENSOR SURFACE

SURFACE DE CAPTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2018 LU 100993**

(43) Veröffentlichungstag der Anmeldung:
**15.09.2021 Patentblatt 2021/37**

(73) Patentinhaber: **Visseiro GmbH**
**10625 Berlin (DE)**

(72) Erfinder:
• **JURASCH, Janek**
**13627 Berlin (DE)**
• **KELBEL, Pirmin**
**10178 Berlin (DE)**

(74) Vertreter: **Remus, Alvaro Johannes**
**BPSH Schrooten Haber Remus**
**Patent- und Rechtsanwaltspartnerschaft mbB**
**Grafenberger Allee 277-287, Eingang A**
**40237 Düsseldorf (DE)**

(56) Entgegenhaltungen:
EP-A1- 1 125 975    WO-A1-2016/053398
WO-A1-2018/009165    WO-A2-2017/078822
US-A1- 2015 230 747

• CHUO Y ET AL: "Sensor Layer of a Multiparameter Single-Point Integrated System", IEEE TRANSACTIONS ON BIOMEDICAL CIRCUITS AND SYSTEMS, IEEE, US, Bd. 3, Nr. 4, 1. August 2009 (2009-08-01), Seiten 229-240, XP011327556, ISSN: 1932-4545, DOI: 10.1109/TBCAS.2009.2021769
• HWANG SU HWAN ET AL: "Unconstrained Sleep Apnea Monitoring Using Polyvinylidene Fluoride Film-Based Sensor", IEEE TRANSACTIONS ON BIOMEDICAL ENGINEERING, IEEE SERVICE CENTER, PISCATAWAY, NJ, USA, Bd. 61, Nr. 7, 1. Juli 2014 (2014-07-01), Seiten 2125-2134, XP011551243, ISSN: 0018-9294, DOI: 10.1109/TBME.2014.2314452 [gefunden am 2014-06-16]
• Onda Corp.: "Acoustic Properties of Rubbers Ref Material Vendor V L mm/:s", , 11. April 2003 (2003-04-11), XP055415635, Gefunden im Internet: URL:http://www.ondacorp.com/images/Rubbers .pdf [gefunden am 2017-10-13]
• M Acetate: "Acoustic Properties of Liquids Ref Liquid Vendor V L mm/:s", , 11. April 2003 (2003-04-11), Seite 5, XP055653830, Gefunden im Internet: URL:http://www.ondacorp.com/images/Liquids .pdf [gefunden am 2019-12-18]

**Beschreibung**

Gebiet der Erfindung

Die Erfindung betrifft eine Vorrichtung zum Erfassen von mechanischen Schwingungen menschlichen oder tierischen Gewebes, welche mindestens einen Sensor umfasst, der in ein viskoelastisches Medium eingebettet ist, wobei der Sensor und das viskoelastische Medium eine Sensorik bilden und das viskoelastische Medium eine Oberfläche für den Kontakt zum menschlichen oder tierischen Gewebe bildet, wobei das viskoelastische Medium eine Schallkennimpedanz zwischen 1400 bis 2000 kNs/m$^3$ aufweist, und wobei ein Teil der Oberfläche des viskoelastischen Mediums mit einem angrenzenden schallreflektierenden Material bedeckt ist. Die Erfindung betrifft ferner ein Verfahren zur Herstellung dieser Vorrichtung, bei dem mindestens ein Sensor in ein viskoelastisches Medium eingebettet wird, wobei das viskoelastische Medium eine Schallkennimpedanz zwischen 1400 bis 2000 kNs/m$^3$ aufweist. Die Vorrichtung kann im medizinischen Bereich sowie im persönlichen Gesundheitsbereich eingesetzt werden. Anwendungsgebiete finden sich im Medizintechnik- und Lifestylebereich.

Hintergrund der Erfindung

Bei der Ballistokardiographie werden alle Bewegungen (und mechanischen Schwingungen) im menschlichen Gewebe erfasst. Das gewonnene Rohsignal enthält Informationen über den Herzschlag, die Atmung und alle weiteren mechanischen Bewegungen, welche die Person durchführt. Damit einzelne Aussagen bzgl. der jeweiligen Parameter möglich sind, müssen die Signalkomponenten getrennt werden. Durch die unterschiedlichen Frequenzbereiche der Signalanteile kann das Signal mit Hoch- und Tiefpassfiltern elektrisch separiert werden und die Parameter können einzeln erfasst werden. Diese Signalverarbeitung erfolgt durch Hard- und Software.

Es sind bereits eine Vielzahl an Messgeräten und Produkten zur Messung von Parametern auf Basis der Ballistokardiographie (BKG) im Stand der Technik bekannt. Diese basieren auf unterschiedlichen Sensoren und Aufbauten für verschiedene Anwendungen. Die verschiedenen Sensoren umfassen unter anderem: Piezoaktive Materialien, differenzieller Luft- oder Wasserdruck und Hochfrequenzradar.

Die publizierte internationale Patentanmeldung WO 2017/134681 A2 offenbart ein System für BKG-Messungen unter Verwendung von Piezosensoren mit dem Ansatz die Sensoren mittels einer festen Platte aus Holz oder ähnlichem Material zu verbinden, um die Fläche zu vergrößern. Die Sensoren werden auf dem Material angebracht. Es werden explizite Größenverhältnisse genannt. Nachteilig ist bei dieser Offenbarung, dass der Sensor auf der Oberfläche der Platte angeordnet ist.

In der publizierten internationalen Patentanmeldung WO 2007/061619 A1 wird ein piezoaktiver Sensor beschrieben, der als Ausleger (Cantilever) frei in der Luft schwingen kann. Dabei ist eine zusätzliche Masse vorgesehen, um die Sensitivität zu steigern. Nachteilig an dieser Offenbarung ist, der komplexe Aufbau mit einem gesonderten Ausleger und das benötigte feste strukturelle Gehäuse, welches den Cantilever umschließen muss.

Die publizierte deutsche Patentanmeldung DE 10 2013 110 900 A1 beschreibt das Konzept der Schallkennimpedanzanpassung für Ultraschallanwendung unter Verwendung einer oder mehrerer piezobasierter Sensoren und Luftankopplung. Nachteilig an dieser Offenbarung ist, dass zur Oberflächenvergrößerung der gesamte Sensor vergrößert werden muss.

Die publizierte internationale Patentanmeldung WO 2016/053398 A1 offenbart ein Sitzkissen mit fiberoptischen Sensoren zur Erfassung von Vitalparametern. Zusätzlich sind mehrere Drucksensoren eingebaut, die die Druckverteilung beim Sitzen messen. Die Sensorik ist in mehreren Lagen aufgebaut und in einem elastischen Medium eingebettet, welches ein Kissen darstellt. Elektronik und Batterie sind ebenfalls enthalten. Das elastische Medium kann ein Schaummaterial sein und dient ausschließlich dem Sitzkomfort.

Aus der WO 2018/009165 A1 ist ein tragbarer Blutdruckmonitor bekannt, der ein dehnbares Substrat umfasst, in den ein Halbleiter-Chip eingebettet ist. Der Chip ist elektrisch mit Sensoren gekoppelt, die kardiographische Signale erfassen können.

Chuo and Kaminska (IEEE Transactions on Biomedical Circuits and Systems, Vol. 3, No. 4, Aug. 2009) beschreiben einen tragbaren medizinischen Sensor, der ein biegsames, mehrschichtiges Polymersubstrat umfasst, in das mehrere Sensoren eingebettet sind, die unterschiedliche kardiographische Signale erfassen können.

Aus der US 2015/230747 A1 ist ferner ein Verfahren und System zur kontinuierlichen und kontaktlosen Messung von Gewebekompressionen bekannt, bei dem die Verzerrung ballistischer Signale, die beim Durchfließen menschlicher Gewebe entsteht, mittels eines Sensorelements erfasst werden.

Ein wesentlicher Nachteil aller im Stand der Technik beschriebenen Systeme besteht darin, dass die Fläche, über die die Schwingungen erfasst werden können, von der Fläche der jeweiligen Sensoren abhängt. Wenn also große Messflächen benötigt werden, müssen auch die Sensoren entsprechend großflächig ausgebildet sein. Diese werden dadurch anfälliger gegenüber Beschädigungen und ferner werden die Kosten für die Herstellung der Systeme erheblich erhöht.

Aufgabe der Erfindung

Es ist die Aufgabe der vorliegenden Erfindung, eine Vorrichtung der eingangs genannten Art zur Verfügung zu stellen, welche die Nachteile der aus dem Stand der Technik bekannten Lösungen vermeidet.

Zusammenfassung der Erfindung

Die Aufgabe wird durch eine Vorrichtung der eingangs genannten Art gelöst, bei der ein Teil der Oberfläche des viskoelastischen Mediums mit einem angrenzenden Material bedeckt ist, wobei das angrenzende Material nur noch eine Seite des viskoelastischen Mediums für den Kontakt mit dem menschlichen oder tierischen Gewebe frei lässt. Die Vorrichtung umfasst erfindungsgemäß eine Sensorik, die im Wesentlichen von einem viskoelastischen Medium (z.B. Gel) gebildet wird, das die zu erfassenden Schwingungen aufnimmt und an mindestens einen Sensor (Schwingungswandler) weiterleitet. Das viskoelastische Medium ist also erfindungsgemäß Teil der Sensorik und stellt dabei mit seiner Oberfläche die aktive Messfläche zur Verfügung. Der Kontakt zum menschlichen oder tierischen Gewebe kann dabei direkt und/oder indirekt, d. h. beispielsweise über eine Folie, ein Kleidungsstück, sonstige Textilien oder Ähnliches, hergestellt werden. Darüber hinaus werden die Schwingungen (z.B. Schallwellen) durch das viskoelastische Medium geleitet, so dass nicht die gesamte Vorrichtung bzw. Sensorik in Schwingung gebracht werden muss. Das hat den Vorteil, dass bei gleichem Signalqualität- und Flächenverhältnis weniger Energie benötigt wird. Diese vorteilhaften Eigenschaften werden durch die Einstellung der mechanischen Transmissionseigenschaften des Gels ermöglicht, d. h. insbesondere dadurch, dass die Schallkennimpedanz des viskoelastischen Mediums an menschliche oder tierische Körper angepasst ist. Erfindungsgemäß muss das viskoelastische Medium also eine spezifische, der Anwendung entsprechende Schallkennimpedanz aufweisen. Die Schallkennimpedanz kann unter anderem durch das Kompressionsmodul, die Dichte und das Schubmodul eingestellt bzw. bestimmt werden. Der Sensor dient innerhalb der Sensorik ausschließlich als Schwingungswandler, so dass die Messfläche und die Qualität/Sensitivität der erfindungsgemäßen Vorrichtung nicht von der Größe bzw. Fläche des Sensors abhängt. Es spielt dabei keine Rolle, an welcher Stelle der Sensor in das viskoelastische Madium eingebettet ist. Daher kann die Oberfläche des Sensors deutlich kleiner als die empfindliche Oberfläche der gesamten Sensorik bzw. des viskoelastischen Mediums sein. D. h., das viskoelastische Medium bildet die (große) sensitive Oberfläche für den Kontakt zum menschlichen oder tierischen Gewebe, während nur eine deutlich kleinere Fläche für den Sensor ausreichend ist. Dadurch können die Herstellungskosten für die erfindungsgemäße Vorrichtung erheblich gesenkt werden. Insbesondere können auch große Messflächen kostengünstig hergestellt werden.

In einer beispielhaften Ausführungsform der Erfindung kann es sich bei dem Sensor in vorteilhafter Weise um einen Drucksensor handeln. Beispielsweise kann der Sensor aus einem piezoaktiven Material bestehen.

In einer weiteren beispielhaften Ausführungsform der Erfindung ist vorgesehen, dass der Sensor flächig und beidseitig signalempfindlich ist. Dies ist insbesondere dann von Vorteil, wenn der Sensor freischwimmend in das viskoelastische Medium eingebettet ist.

Beispielsweise kann der Sensor eine Dicke von weniger als 1 mm aufweisen und/oder eine Fläche von kleiner 100 cm$^2$ haben.

In einer weiteren beispielhaften Ausführungsform der Erfindung ist vorgesehen, dass zumindest eine Seite der Sensorik derart ausgebildet ist, dass mit dieser Seite ein flächiger Kontakt mit dem menschlichen oder tierischen Gewebe herstellbar ist. Zumindest eine Seite der Sensorik kann also so geformt sein, dass mit dieser Seite ein groß- oder ganzflächiger Kontakt mit dem menschlichen oder tierischen Gewebe hergestellt werden kann.

Die Sensorik kann beispielsweise eine Dicke von bis zu 100 mm aufweisen.

In vorteilhafter Ausgestaltung der Erfindung ist ferner vorgesehen, dass das viskoelastische Medium aus einem homogenen Material besteht. Das viskoelastische Gel sollte homogen sein, um die oben beschriebene Schwingungsübertragung (z.B. Schalltransmission) zu erreichen. Insbesondere darf es nicht heterogen sein (wie beispielsweise geschäumte Materialien), da es an den Grenzschichten zu Inhomogenitäten kommt, welche die Signalqualität negativ beeinflussen bzw. stören. "Homogen" im Sinne der Erfindung bedeutet daher, dass das entsprechende Material keine Partikel, Einschlüsse, Gasbläschen, Flüssigkeitströpfchen, Klumpen, Verunreinigungen oder sonstige die Homogenität störende Elemente enthält, welche die Weiterleitung von Schwingungen (z.B. Schallwellen) beeinträchtigen könnten.

In einer weiteren beispielhaften Ausführungsform der Erfindung ist vorgesehen, dass das viskoelastische Medium eine Dichte im Bereich von 900 bis 1100 kg/m$^3$ und eine Härte von 15 shore A bis 40 shore A aufweist. Diese Eigenschaften des viskoelastischen Mediums, vorzugsweise eines Gels, sind in vorteilhafter Weise an Vitalparameter und eine Kontaktierung des menschlichen oder tierischen Gewebes beispielsweise durch Kleidung hindurch angepasst.

Beispielsweise kann es sich bei dem viskoelastischen Medium um Komponenten aus den Gruppen der Carbamate, Polyole, Polyisocyanate oder Silikone handeln, wobei das Medium wasserfrei sein kann.

In einer weiteren beispielhaften Ausführungsform ist vorgesehen, dass das Flächenverhältnis zwischen Sensorfläche und Kontaktfläche der Sensorik maximal 1:4 ist. Die Oberfläche des Sensors (Schwingungswandlers) ist erfindungsgemäß also deutlich kleiner als die empfindliche Oberfläche der gesamten Sensorik bzw. die Oberfläche des viskoelastischen Mediums. D. h., das viskoelastische Medium bildet eine große Oberfläche, während nur eine relativ kleine Fläche für den Sensor benötigt wird. Das Größenverhältnis zwischen Sensoroberfläche und der Oberfläche des viskoelastischen Mediums kann beispielsweise mindestens 4:1, vorzugsweise 5:1, besonders bevorzugt 10:1, weiter bevorzugt 25:1, insbesondere 50:1, betragen.

In einer weiteren beispielhaften Ausführungsform der Erfindung ist zudem vorgesehen, dass der zumindest eine Sensor eine Sendeeinheit umfassen kann. In einer weiteren Ausführungsform kann der Sensor beispielsweise über Kabel oder drahtlos mit einer Auswerteeinheit verbunden sein.

Weiterhin kann das viskoelastische Medium zum Schutz mit einer Polyurethanfolie und/oder Textilien umschlossen sein.

In einer weiteren beispielhaften Ausführungsform ist vorgesehen, dass die Schallkennimpedanzen des viskoelastischen Mediums und des angrenzenden Materials an deren Grenzschicht entsprechend der Ungleichung

$$\left| \frac{Z_{M3} - Z_{M2}}{Z_{M3} + Z_{M2}} \right| > 0{,}85$$

mit

$Z_{M2}$ = Schallkennimpedanz des viskoelastischen Mediums und

$Z_{M3}$ = Schallkennimpedanz des angrenzenden Materials

eingestellt sind. Durch diese Ausgestaltung der erfindungsgemäßen Vorrichtung wird eine optimale Entkopplung der Sensorik in Bezug auf die Umgebung bzw. störende Einflüsse aus der Umgebung gewährleistet. Die Entkopplung wird dabei über die Schallkennimpedanz der Materialien an der Grenzschicht zwischen dem viskoelastischen Medium und dem angrenzenden Material erreicht. Abhängig vom Anwendungsfall kann die Sensorik zu diesem Zweck beispielsweise mit einem Schaumstoff umgeben sein, so dass nur eine Kontaktoberfläche der Sensorik in Kontakt zum menschlichen oder tierischen Gewebe steht. Der Schaumstoff dient der Entkopplung des Messsystems gegenüber Störgrößen von außen, da mechanische Schwingungen nahezu vollständig durch den Schaumstoff gedämpft werden. Das angrenzende Material kann die Sensorik beispielsweise derart umgeben bzw. auf deren Oberfläche aufgebracht sein, dass nur eine Seite bzw. Teilfläche für den Kontakt zum menschlichen oder tierischen Körper unbedeckt bzw. frei bleibt, während alle anderen Seiten bzw. Teilflächen vollständig von dem angrenzenden Material bedeckt sind.

Die Aufgabe wird erfindungsgemäß ferner durch ein Verfahren eingangs genannten Art gelöst, bei dem ein Teil der Oberfläche des viskoelastischen Mediums derart mit einem angrenzenden Material bedeckt wird, dass das angrenzende Material nur noch eine Seite des viskoelastischen Mediums für den Kontakt mit dem menschlichen oder tierischen Gewebe frei lässt.

In vorteilhafter Ausgestaltung des erfindungsgemäßen Verfahrens ist vorgesehen, dass der Sensor freischwimmend in das viskoelastische Medium eingebettet wird, so dass er am Ende des Herstellungsprozesses vollständig vom viskoelastischen Medium umgeben ist.

Insbesondere kann das viskoelastische Medium derart ausgewählt und/oder eingestellt werden, dass es eine Dichte im Bereich von 900 bis 1100 kg/m$^3$ und eine Härte von 15 shore A bis 40 shore A aufweist.

Das Größenverhältnis zwischen Sensoroberfläche und der Oberfläche des viskoelastischen Mediums kann beispielsweise derart ausgestaltet werden, dass es mindestens 4:1, vorzugsweise 5:1, besonders bevorzugt 10:1, weiter bevorzugt 25:1, insbesondere 50:1, beträgt. Die Oberfläche des Sensors (Schwingungswandlers) kann in vorteilhafter Weise also deutlich kleiner gehalten werden als die empfindliche Oberfläche der gesamten Sensorik bzw. die Oberfläche des viskoelastischen Mediums. Dadurch können die Herstellungskosten für die erfindungsgemäße Vorrichtung erheblich gesenkt werden. Insbesondere können auch große Messflächen kostengünstig hergestellt werden.

In einer beispielhaften Ausführungsform des erfindungsgemäßen Herstellungsverfahrens ist in vorteilhafter Weise vorgesehen, dass die Schallkennimpedanzen des viskoelastischen Mediums und des angrenzenden Materials an deren Grenzschicht entsprechend der Ungleichung

$$\left| \frac{Z_{M3} - Z_{M2}}{Z_{M3} + Z_{M2}} \right| > 0{,}85$$

mit

$Z_{M2}$ = Schallkennimpedanz des viskoelastischen Mediums und

$Z_{M3}$ = Schallkennimpedanz des angrenzenden Materials

eingestellt werden. Durch diese Ausgestaltung des erfindungsgemäßen Verfahrens wird eine optimale Entkopplung der Vorrichtung in Bezug auf die Umgebung bzw. störende Einflüsse aus der Umgebung erzielt. Beispielsweise kann die Sensorik zu diesem Zweck derart teilweise mit einem Schaumstoff ummantelt werden, dass nur eine Kontaktoberfläche der Sensorik in Kontakt zum Messobjekt (menschliches oder tierisches Gewebe) stehen kann.

Ein weiterer Gegenstand der vorliegenden Erfindung ist eine Sitz- oder Liegefläche bzw. eine Einrichtung oder ein Möbelstück mit einer Sitz- oder Liegefläche, die eine Vorrichtung wie zuvor beschrieben umfasst.

Die erfindungsgemäße Vorrichtung wie zuvor beschrieben kann in vorteilhafter Weise zur Erfassung von mechanischen Schwingungen menschlicher oder tierischer Körper verwendet werden, insbesondere im medizinischen Bereich und im persönlichen Gesundheitsbereich sowie im Medizintechnik- und Lifestylebereich.

Weitere Aspekte, Merkmale und Vorteile der vorliegenden Erfindung ergeben sich ohne weiteres aus der folgenden detaillierten Beschreibung, in der einfach bevorzugte Ausführungsformen und Implementierungen dargestellt sind. Die vorliegende Erfindung kann auch in anderen und unterschiedlichen Ausführungsformen verwirklicht werden und ihre verschiedenen Details können in verschiedenen, offensichtlichen Aspekten modifiziert werden, ohne Lehre und Umfang der vorliegenden Erfindung zu verlassen. Dementsprechend sind die Zeichnungen und Beschreibungen als veranschaulichend und nicht als einschränkend anzusehen. Zusätzliche Aufgaben und Vorteile der Erfindung werden teilweise in der folgenden Beschreibung dargelegt und werden teilweise aus der Beschreibung offensichtlich oder können der Ausführung der Erfindung entnommen werden.

Zusammenfassung der Figuren

Die vorliegende Erfindung wird anhand von Figuren und Ausführungsbeispielen beschrieben und dargestellt. Für den zuständigen Fachmann ist klar, dass die Erfindung nicht auf den Inhalt der Figuren und Ausführungsbeispiele beschränkt ist. Es zeigt:

FIG. 1    Gesamtsystem bestehend aus der Sensorik/ Sitzauflage (100 und 101), der Auswertebox (103) und dem Verbindungskabel (102)

FIG. 2A-G    mögliche Formen der Sensorik. Diese sind nur Beispiele und stellen keine vollständige Liste aller möglichen Formen dar

FIG. 3    Auswirkungen mechanischer Lasten auf die piezoaktive Sensorfolie (101) bei verschiedenen Ausführungen

FIG. 4    Darstellung der Signalverarbeitung

FIG. 5    Funktionsprinzip der Schallkennimpedanzanpassung

Ausführliche Beschreibung der Erfindung

Die zuvor formulierte Aufgabe der Erfindung wird durch die Merkmale der unabhängigen Ansprüche gelöst. Die anhängigen Ansprüche decken weitere spezifische Ausführungsformen der Erfindung ab.

Die vorliegende Erfindung betrifft eine Sensoroberfläche bzw. den Aufbau einer Sensorik und basiert auf dem Einbetten von kleinflächigen, piezoaktiven Sensorfolien (PVDF-Folie, Piezosensoren, Sensorfläche) in ein viskoelastisches Medium. Die Sensoroberfläche /Sensorik kann für ein Gerät verwendet werden, welches als eigenständige Sensorik oder als integriertes Modul, beispielsweise in verschiedene Sitz- und Liegemöbel integriert werden kann. Die Sensorik ist speziell für Messungen an Lebewesen (Mensch, Tier) angepasst. Dabei nimmt die Sensorik hautkontaktlos alle mechanischen Schwingungen, insbesondere ballistokardiographische Signale auf. Es werden insbesondere Parameter wie beispielsweise Bewegung, Atmung (Atemfrequenz, Atemamplitude), Herzfrequenz und Herzratenvariabilität gemessen.

Das Einbetten der Sensorfolien findet während des Herstellungsprozesses statt. Dabei wird das viskoelastische Medium in eine Form gegossen und reagiert dann vollständig aus. Während des Reaktionsprozesses kann die piezoaktive Sensorfolie in den Vergussprozess integriert werden, sodass diese ohne Lufteinschlüsse vollständig von dem Medium umschlossen ist.

Die Neuheit der vorliegenden Erfindung liegt in dem Aufbau unter Berücksichtigung von spezifischen Eigenschaften des viskoelastischen Mediums. Die Schallkennimpedanz des Mediums entspricht im Idealfall der des menschlichen Gewebes (vgl. Tabelle 1). Bei direktem Kontakt zwischen Medium und menschlichem Gewebe werden so nahezu alle mechanischen Schwingungen (Bewegungen im menschlichem Gewebe, akustische Schwingungen) vom menschlichen Gewebe in das Medium transmittiert.

Zusätzliche Materialien, beispielsweise die Kleidung der Person oder Bezüge für die Sensorik, wirken als Brücke (112) bei der Schallübertragung. Da das Material der Brücke nicht homogen ist, ist keine Schallkennimpedanz bestimmbar. Eine Einschränkung für die Brücke ergibt sich aus dem jeweiligen Anwendungsfall. Bei der Anwendung wird das Brücken-Material (beispielsweise Baumwoll-Gewebe) durch die wirkenden Kräfte komprimiert. Unter der Idealisierung der Brücke ermittelt sich der Transmissions-, bzw. Reflexionsfaktor zwischen viskoelastischen Medium (100) und menschlichem (oder tierischem) Gewebe (111) zu:

$$\text{Reflexionsfaktor: } r = \frac{Z_2 - Z_1}{Z_2 + Z_1}, \quad |r| \leq 1$$

$$\text{Transmissionsfaktor: } t = \frac{2 \cdot Z_2}{Z_2 + Z_1} = 1 - |r|$$

wobei $Z_1$ und $Z_2$ den Schallkennimpedanzen zweier angrenzender Materialien entsprechen

Tabelle 1: Schallkennimpedanzen und Dichte ausgewählter Materialien und Strukturen

| Material | Schallkennimpedanz Z in kNs/m$^3$ | Dichte in kg/m$^3$ |
|---|---|---|
| Wasser (30°C) | 1502 | 1000 |
| Luft(20°C) | 0,4136 | 1,2 |
| Menschliches Gewebe (Durchschnittlich) | 1573 | 940 - 1040 |
| viskoelastisches Medium | 1400 - 2000 | 900 - 1100 |

Mit den Werten aus Tabelle 1 ergeben sich die durchschnittlichen Transmissions- und Reflexionsfaktoren in Tabelle 2 zu:

| Grenzschicht: | Reflexionsfaktor [r] | Transmissionsfaktor [t] |
|---|---|---|
| Gewebe - Luft | -0,9995 | 0,0005 |
| Medium - Luft | -0,9996 | 0,0004 |
| Gewebe - Medium | -0,1195 | 0,8805 |

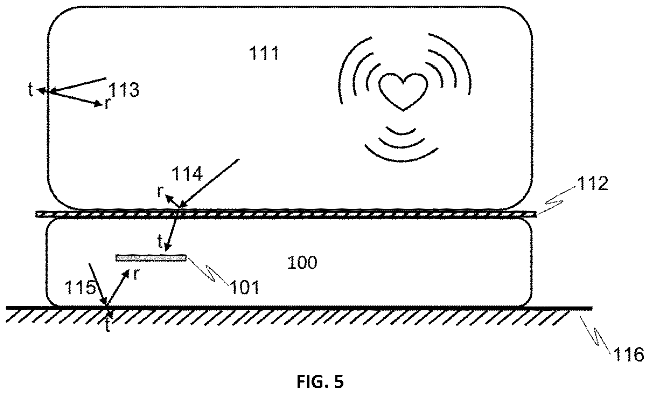

Die von menschlichem Gewebe erzeugten Schwingungen werden an der Grenzfläche zur Luft reflektiert und zu einem Großteil in das Medium transmittiert. FIG. 5 stellt dieses Prinzip schematisch dar. Ein Vorteil dieser Erfindung liegt darin, dass durch die Kopplung und mehrfache Reflektion der Schwingungen in dem Medium die benötigte piezoaktive Sensorfläche erheblich verkleinert werden kann. So werden bei einer Anwendung der Sensoroberfläche als Sitzkissen nur wenige Quadratzentimeter piezoaktiver Sensorfolie benötigt.

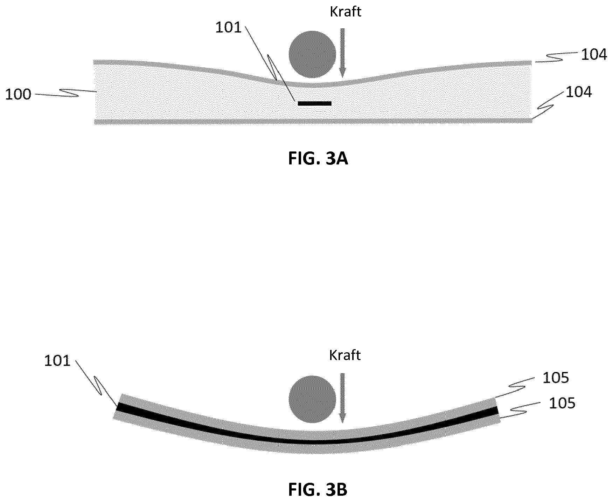

Durch das Medium wird gleichzeitig die Oberfläche für den mechanischer Kontakt zwischen menschlichem Gewebe und Sensorik hergestellt. Die Oberfläche der Sensorik ist flexibel und verformbar. Das Medium schützt die piezoaktiven Sensorfolien vor großen mechanischen Belastungen (vgl. FIG. 3). Dadurch wird der mechanische Verschleiß der piezoaktiven Sensorfolien gesenkt und somit die Lebensdauer erhöht.

Die einzustellenden Eigenschaften insbesondere bezüglich der physikalischen Dichte und der Schallkennimpedanz des viskoelastischen Mediums ergeben sich aus dessen Zusammensetzung bei der Herstellung. Die Herstellung und Zusammensetzung eines viskoelastischen Mediums ist bereits bekannt, beispielsweise die Zusammensetzung und die Herstellung eines viskoelastisches Mediums auf Basis von Reaktionsprodukten aus Polyolen und Polyisocyanaten (EP 1125975B1).

Die Erfindung wird anhand der dargestellten Abbildungen beschrieben. Die Figuren zeigen ein erfindungsgemäßes Messgerät, das alle Bewegungen, mechanischen Schwingungen und somit auch akustische Schwingungen innerhalb des Gewebes eines Lebewesens (Mensch oder Tier) aufzeichnet. Dabei wird es dazu genutzt die Vitalität des Lebewesens festzustellen und zu erfassen. Die abgeleiteten Größen sind mindestens: Bewegung, Herzfrequenz, Herzratenvariabilität, Atemfrequenz, Atemamplitude.

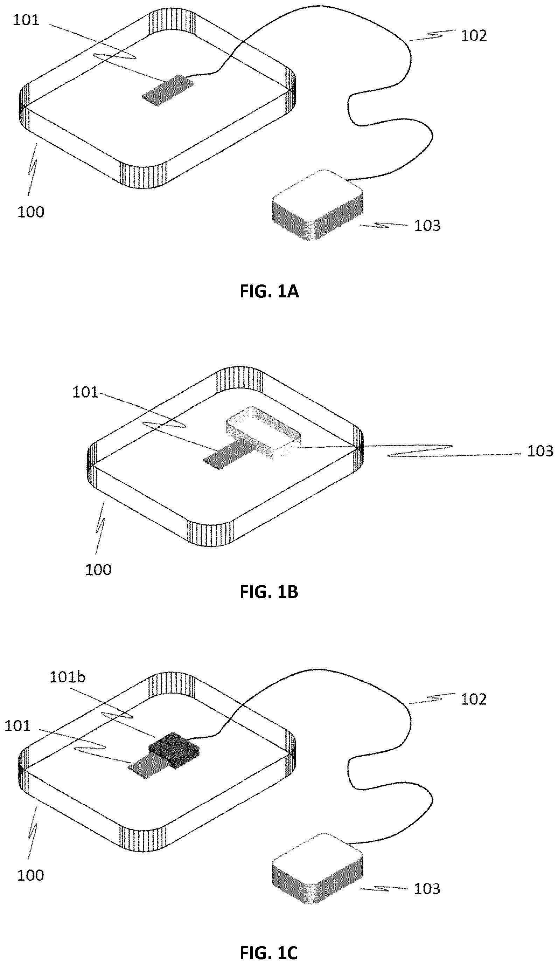

FIG. 1 zeigt schematisch den gesamten Aufbau des Sensorsystems. Dieses besteht aus der Sensorik (100 und 101), einem Verbindungskabel (102) und der Auswerteeinheit (103). Die Energieversorgung der Auswerteeinheit (103) ist nicht dargestellt. Diese kann über eine integrierte Energieversorgung (Batterie, Akku) oder über ein externes Netzgerät erfolgen. Im Falle der Versorgung über ein externes Netzgerät wird dieses ebenfalls per Kabel an die Auswerteeinheit angeschlossen. Die gesamte Auswerteelektronik (103) kann dabei ganz oder teilweise in die Sensorik (100 und 101) integriert sein (vgl. FIG. 1b).

Weiterhin ist in FIG. 1 der Aufbau der Sensorik (100 und 101) dargestellt. Die Sensorik besteht aus einem viskoelastischen Medium, welches beispielsweise Technogel (BTGS, ummantelt mit PU Folie (104, FIG. 3) sein kann. Entscheidende Eigenschaften des Mediums sind die mechanischen Eigenschaften Dichte und Härte.

Innerhalb des viskoelastischen Mediums werden eine oder mehrere piezoaktive Sensorfolien (101) eingebracht. In FIG. 1 ist nur eine piezoaktive Sensorfolie dargestellt, es können auch mehrere piezoaktive Sensorfolien eingebaut werden, dies kann weitere Vorteile haben (Signalkorrelation). Zusätzlich kann eine elektrische Verstärkerschaltung (101b), direkt am Sensor (101) oder zumindest innerhalb des viskoelastischen Mediums (100) eingebracht sein und mit der piezoaktiven Sensorfolie verbunden sein. Diese dient der Signalverstärkung, bzw. der Reduzierung von (elektrischen) Störgeräuschen und der Anfälligkeit des Signals bzgl. (elektrischen) Störgeräuschen, durch eine Impedanzanpassung (vgl. FIG. 1C).

Die Verwendung des viskoelastischen Mediums erfüllt mehrere Zwecke: Durch die viskosen Eigenschaften werden lokal einwirkende Kräfte verteilt und die Belastung auf die piezoaktive Sensorfolie (101) reduziert. In FIG. 3 sind dazu zwei schematische Aufbauten skizziert. FIG. 3A entspricht dem Aufbau der Sensorik aus FIG. 1 in einer Seitenansicht. Eine auftreffende Kraft, beispielsweise eine auf der Sensorik sitzende oder liegende Person, verformt primär das viskoelastische Medium: Die piezoaktive Sensorfolie (101) wird reduziert verformt (Senkung durch mechanischen Stress).

FIG. 3B zeigt den Stand der Technik für den Aufbau einer solchen Sensorik für ballistokardiographische Messungen. Die piezoaktive Sensorfolie füllt die gesamte Dicke und Breite der Sensorik aus und ist nur von einer dünnen Deckschicht (105) ohne dämpfende Eigenschaften (Folie, Kunststoffschicht) umgeben. Dadurch hat eine auftretende Kraft eine direkte mechanische Belastung (Stress) auf die piezoaktive Sensorfolie zur Folge. Insbesondere mechanische Verformungen der Folie haben eine starke Alterung dieser zur Folge.

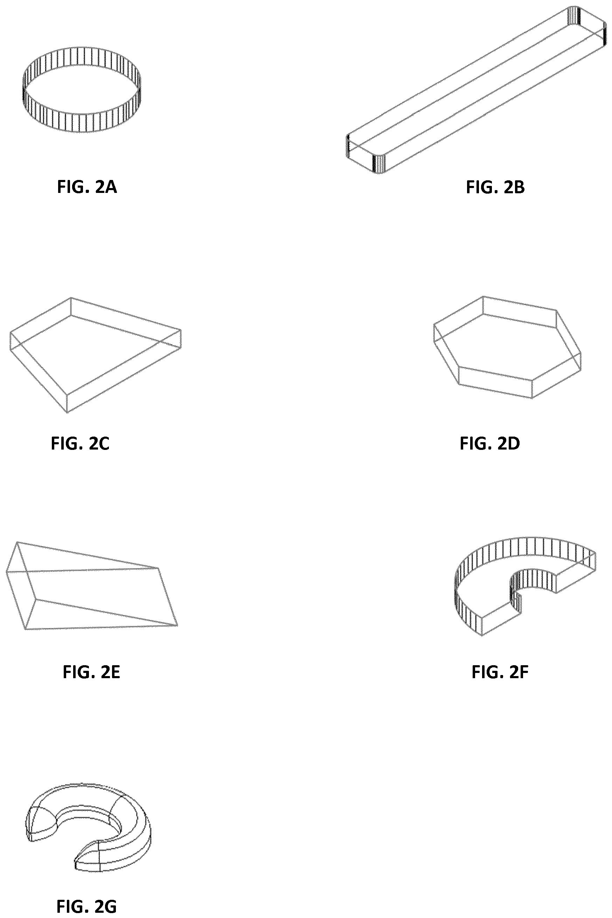

Das viskoelastische Medium gemäß der vorliegenden Erfindung ist verformbar. Es ist formstabil bei Belastung, z.B. bis zu einer achtfachen Ausdehnung dehnbar, und kann bei der Herstellung in eine beliebige dreidimensionale Form gegossen werden. Durch die viskoelastischen Eigenschaften des Materials der vorliegenden Erfindung kann sich die Sensorik an eine Form, beispielsweise die Sitzschale eines Stuhls, anpassen. Dabei wird piezoaktive Sensorfolie marginal verformt und somit mechanisch belastet. Mögliche dreidimensionale Formen der gesamten Sensorik sind beispielhaft in FIG. 2A - 2G dargestellt. Wobei die dargestellten Formen keine abschließende Liste möglicher Formen darstellen.

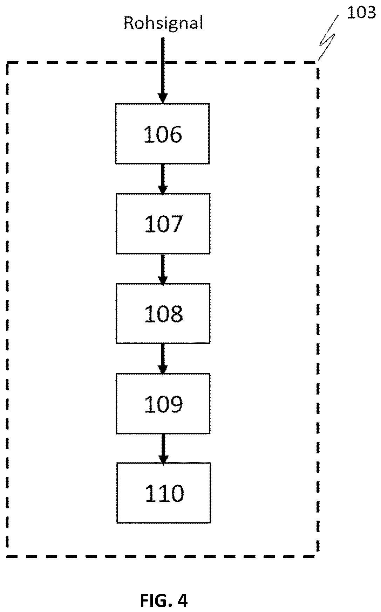

In FIG. 4 ist der schematische Ablauf der Signalverarbeitung dargestellt. Das eintreffende Rohsignal wird zunächst verstärkt (106). Dazu sind in dem Fachmann aus dem Stand der Technik verschiedene Schaltungen bekannt.

Nach der Signalverstärkung wird das Signal mit Hoch- und Tiefpassfiltern gefiltert (107). Auch deren Design und Aufbau sind dem Fachmann bekannt und müssen für die neue Sensorik ausgelegt werden. In 108 wird das Signal digitalisiert und an einen digitalen Signalprozessor weitergeleitet (109). Innerhalb des digitalen Signalprozessors wird das Signal analysiert und die Parameter wie Bewegung, Herzfrequenz, Atemfrequenz und Herzratenvariabilität werden extrahiert und berechnet, z.B. durch digitale Filter.

Zur Auswertung der Rohsignale der Sensorik ist eine Auswerteeinheit mit Hard- und Softwarekomponenten notwendig. Auswerteeinheit und Sensorik sind ein Sensorsystem. Die der Sensorik nachgeschaltete Hardware (103) und Software dient der Signalverarbeitung und Auswertung der ballistokardiographischen Rohsignale. In einer möglichen Anwendung werden die Parameter extrahiert und auf Basis der gewonnen Parameter weitere Bewertungsgrößen abgeleitet. In einer erfindungsgemäßen Ausführungsform können diese Daten über eine drahtlose Funkverbindung an ein mobiles Endgerät, Cloud-Dienst, Server, Webinterface, Weiterverarbeitung (...) gesendet oder über eine Anzeige (Display) direkt am Gerät angezeigt werden.

Zusätzlich können die erfassten einzelnen Parameter auch kombiniert werden, um weitere medizinisch relevante Aussagen abzuleiten. Beispielsweise einen ganzheitlichen Gesundheits- oder Fitnessstatus. Anschließend werden die Daten dem Nutzer zur Verfügung gestellt (110). Dazu kann eine Anzeige (Display) direkt an der Auswerteeinheit angebracht werden oder die Daten werden per Funkverbindung oder Kabelgebunden an weitere externe Anzeigegeräte, Speichermedien oder Server übermittelt und eventuell gespeichert.

FIG. 5 zeigt das schematische Funktionsprinzip einer erfindungsgemäßen Sensorik. Vom Herzen (oder anderen Quellen) erzeugte Schwingungen durchlaufen das menschliche Gewebe und treffen auf die Grenzflächen des menschlichen Gewebes. Bei der Grenzfläche zu Luft (113) wird der Großteil der Schwingung reflektiert ("r"). Bei der Grenzfläche (114) zur Sensorik bzw. dem viskoelastischem Medium (100) wird der Großteil der Schwingung transmittiert und trifft innerhalb des Mediums auf die im Verhältnis zur Grenzfläche kleine piezoaktive Sensorfolie (101). An der Grenzfläche (115) zwischen angrenzendem Material (116) und viskoelastischem Medium (100) wird der Großteil der Schwingung wieder reflektiert und trifft erneut auf die piezoaktive Sensorfolie (101) (beidseitig). Zu beachten ist dabei, dass eine Impedanzanpassung zwischen der Unterseite des Mediums (100) und dem angrenzenden Material (116) nicht gegeben sein darf. Dabei müssen die Schallkennimpedanzen folgender Ungleichung genügen:

$$\left|\frac{Z_{M3} - Z_{M2}}{Z_{M3} + Z_{M2}}\right| > 0,85$$

$Z_{M2}$ enspricht der Schallkennimpedanz des viskoelastischen Mediums (100)
$Z_{M3}$ enspricht der Schallkennimpedanz des angrenzenden Materials (116)

Da die Sensorik bzw. das viskoelastische Medium (100) in alle Richtungen sensitiv ist, muss diese, um ein nutzbares Signal-to-Noise Verhältnis zu erhalten, von der Umgebung entkoppelt werden und darf nur Kontakt mit der Signalquelle (menschliches oder tierisches Gewebe 111) haben. Um diese Entkopplung durchzuführen, kann das viskoelastische Medium (100) beispielsweise in eine Schaumstoffwanne (angrenzendes Material 116) eingebettet werden, welche nur noch eine Seite des viskoelastische Mediums (100) für den Kontakt mit der Signalquelle frei lässt. Diese Schaumstoffwanne hat bezüglich des Materials inverse Anforderungen an die Schallkennimpedanz.

Eine Anwendung der vorstehend beschriebenen Erfindung ergibt sich bei der persönlichen Verfolgung von Gesundheitsparametern (Personal Healthtracking) und dem Monitoring des eigenen Lebensstils (Quantify yourself). Die erfindungsgemäße Sensorik kann aber auch als Medizinprodukt eingesetzt werden.

Im Vergleich zu dem in der WO 2017/134681 A2 offenbarten, starren Aufbau ist bei der vorliegenden Erfindung eine Integration der Sensoren in das oberflächenvergrößernde Material gegeben, woraus der Vorteil einer mehrfachen Reflektion von Signalen resultiert. Weiterhin ist eine Sensorfolie der vorliegenden Erfindung beidseitig aktiv, was in einer Verstärkung von Signalen resultiert. Zudem ist die Möglichkeit einer Impedanz-Anpassung durch die Wahl der Materialeigenschaften gegeben. Ein weiterer Vorteil resultiert daraus, dass sich das Material der Oberfläche des menschlichen Gewebes anpassen kann (elastisch).

Im Hinblick auf die Lehre der WO 2007/061619 A1 ist bei der vorliegenden Erfindung die aktive Sensorfläche vollständig von einem Schallkennimpedanz angepassten Medium (viskoelastisches Medium) umschlossen. Luft bietet bei der Lehre nach der WO 2007/061619 keine Möglichkeit zur Impedanzanpassung.

In Bezug auf das in der DE 10 2013 110 900 A1 beschriebene Konzept, erfolgt gemäß der vorliegenden Erfindung die Schallkennimpedanzanpassung zwischen menschlichem Gewebe und der Sensorik. Dadurch kann vorteilhafterweise eine Reduzierung der Sensoroberfläche generiert werden, was eine erhebliche Kostenreduktion zur Folge hat.

Die vorstehende Beschreibung der bevorzugten Ausführungsform der Erfindung wurde zum Zweck der Veranschaulichung und Beschreibung gegeben. Es ist nicht beabsichtigt, erschöpfend zu sein oder die Erfindung genau auf die offenbarte Form zu beschränken. Modifikationen und Variationen sind angesichts der obigen Lehre möglich oder können aus der Praxis der Erfindung erlangt werden. Die Ausführungsform wurde gewählt und beschrieben, um die Prinzipien der Erfindung und ihre praktische Anwendung zu erläutern, um es dem Fachmann zu ermöglichen, die Erfindung in verschiedenen Ausführungsformen zu verwenden, die für die spezielle beabsichtigte Verwendung geeignet sind. Es ist beabsichtigt, dass der Umfang der Erfindung durch die beigefügten Ansprüche definiert wird.

Bezugszeichenliste

| | |
|---|---|
| 100 | Viskoelastisches Medium |
| 101 | Sensor |
| 101b | Verstärkerschaltung |
| 102 | Verbindungskabel |
| 103 | Auswerteeinheit |
| 104 | Deckschicht |
| 105 | Deckschicht als direkte Ummantelung des Sensors |
| 106 | Verstärkung Rohsignal |
| 107 | Filterung Signal |
| 108 | Digitalisierung Signal |
| 109 | Weiterleitung Signal |
| 110 | Zur Verfügung gestelltes Signal |

111     menschliches oder tierisches Gewebe
112     Brücke
113     Grenzfläche Luft
114     Grenzfläche Sensorik
115     Grenzfläche Luft / Medium
116     Angrenzendes Material

**Patentansprüche**

1. Vorrichtung zum Erfassen von mechanischen Schwingungen menschlicher oder tierischer Gewebe (111), welche mindestens einen Sensor (101) umfasst, der in ein viskoelastisches Medium (100) eingebettet ist, wobei der Sensor (101) und das viskoelastische Medium (100) eine Sensorik (100, 101) bilden und das viskoelastische Medium (100) eine Oberfläche für den Kontakt zum menschlichen oder tierischen Gewebe (111) bildet, wobei die Schallkennimpedanz des viskoelastischen Mediums an menschliche oder tierische Körper angepasst ist, und wobei das viskoelastische Medium (100) eine Schallkennimpedanz zwischen 1400 bis 2000 kNs/m$^3$ aufweist, **dadurch gekennzeichnet, dass** ein Teil der Oberfläche des viskoelastischen Mediums (100) mit einem angrenzenden Material (116) bedeckt ist, wobei das angrenzende Material (116) nur noch eine Seite des viskoelastischen Mediums (100) für den Kontakt mit dem menschlichen oder tierischen Gewebe (111) frei lässt, wobei es keine Schallkennimpedanzanpassung zwischen der Unterseite des Mediums und dem angrenzenden Material gibt, so dass ein Großteil der Schwingungen an der Grenzfläche zwischen dem angrenzenden Material und dem Medium reflektiert wird.

2. Vorrichtung nach Anspruch 1, wobei es sich bei dem Sensor (101) um einen Drucksensor handelt.

3. Vorrichtung nach einem der Ansprüche 1 oder 2, wobei der Sensor (101) flächig und beidseitig signalempfindlich ist.

4. Vorrichtung nach einem der Ansprüche 1 bis 3, wobei der Sensor (101) freischwimmend in das viskoelastische Medium (100) eingebettet ist.

5. Vorrichtung nach einem der Ansprüche 1 bis 4, wobei zumindest eine Seite der Sensorik (100, 101) derart ausgebildet ist, dass mit dieser Seite ein flächiger Kontakt mit dem menschlichen oder tierischen Gewebe herstellbar ist.

6. Vorrichtung nach einem der Ansprüche 1 bis 5, wobei das viskoelastische Medium (100) aus einem homogenen Material besteht.

7. Vorrichtung nach einem der Ansprüche 1 bis 6, wobei das viskoelastische Medium (100) eine Dichte im Bereich von 900 bis 1100 kg/m$^3$ und eine Härte von 15 shore A bis 40 shore A aufweist.

8. Vorrichtung nach einem der Ansprüche 1 bis 7, wobei es sich bei dem viskoelastischen Medium (100) um Komponenten aus den Gruppen der Carbamate, Polyole, Polyisocyanate oder Silikone handelt.

9. Vorrichtung nach einem der Ansprüche 1 bis 8, wobei das Flächenverhältnis zwischen Sensorfläche und Kontaktfläche der Sensorik maximal 1:4 ist.

10. Vorrichtung nach einem der Ansprüche 1 bis 9, wobei der mindestens eine Sensor (101) eine Sendeeinheit umfasst.

11. Vorrichtung nach einem der Ansprüche 1 bis 10, wobei das viskoelastische Medium (100) zum Schutz mit einer Polyurethanfolie und/oder Textilien umschlossen ist.

12. Vorrichtung nach einem der Ansprüche 1 bis 11, **dadurch gekennzeichnet, dass** die Schallkennimpedanzen des viskoelastischen Mediums (100) und des angrenzenden Materials (116) an deren Grenzschicht entsprechend der Ungleichung

$$\left| \frac{Z_{M3} - Z_{M2}}{Z_{M3} + Z_{M2}} \right| > 0,85$$

mit

$Z_{M2}$ = Schallkennimpedanz des viskoelastischen Mediums (100) und
$Z_{M3}$ = Schallkennimpedanz des angrenzenden Materials (116) eingestellt sind.

13. Verfahren zur Herstellung einer Vorrichtung zum Erfassen von mechanischen Schwingungen menschlicher oder tierischer Gewebe, bei dem mindestens ein Sensor (101) in ein viskoelastisches Medium (100) eingebettet wird, wobei die Schallkennimpedanz des viskoelastischen Mediums an menschliche oder tierische Körper angepasst ist, und wobei das viskoelastische Medium (100) eine Schallkennimpedanz zwischen 1400 bis 2000 kNs/m$^3$ aufweist, **dadurch gekennzeichnet, dass** ein Teil der Oberfläche des viskoelastischen Mediums (100) derart mit einem angrenzenden Material (116) bedeckt wird, dass das angrenzende Material (116) nur noch eine Seite des viskoelastischen Mediums (100) für den Kontakt mit dem menschlichen oder tierischen Gewebe (111) frei lässt, wobei es keine Schallkenimpedanzanpassung zwischen der Unterseite des Mediums und dem angrenzenden Material gibt, so das ein Großteil der Schwingungen an der Grenzfläche zwischen dem angrenzenden Material und dem Medium reflektiert wird.

14. Verfahren nach Anspruch 13, wobei der Sensor (101) freischwimmend in das viskoelastische Medium (100) eingebettet wird.

**Claims**

1. A device for detecting mechanical vibrations of human or animal tissues (111), comprising at least one sensor (101) embedded in a viscoelastic medium (100), wherein the sensor (101) and the viscoelastic medium (100) form a sensor system (100, 101) and the viscoelastic medium (100) forming a surface for contact with human or animal tissue (111), the characteristic acoustic impedance of the viscoelastic medium being adapted to human or animal bodies, and the viscoelastic medium (100) having a characteristic acoustic impedance of between 1400 and 2000 kNs/m$^3$, **characterized in that** a part of the surface of the viscoelastic medium (100) is covered with an adjacent material (116), the adjacent material (116) leaving only one side of the viscoelastic medium (100) free for contact with the human or animal tissue (111), there being no characteristic acoustic impedance between the underside of the medium and the adjacent material, so that a majority of the vibrations are reflected at the interface between the adjacent material and the medium.

2. The device according to claim 1, wherein the sensor (101) is a pressure sensor.

3. Device according to one of claims 1 or 2, wherein the sensor (101) is planar and signal-sensitive on both sides.

4. Device according to any one of claims 1 to 3, wherein the sensor (101) is embedded in the viscoelastic medium (100) in a free-floating manner.

5. Device according to one of claims 1 to 4, wherein at least one side of the sensor system (100, 101) is designed in such a way that this side can be used to establish planar contact with the human or animal tissue.

6. Device according to any one of claims 1 to 5, wherein the viscoelastic medium (100) consists of a homogeneous material.

7. Device according to any one of claims 1 to 6, wherein the viscoelastic medium (100) has a density in the range of 900 to 1100 kg/m$^3$ and a hardness of 15 shore A to 40 shore A.

8. Device according to any one of claims 1 to 7, wherein the viscoelastic medium (100) is components from the groups of carbamates, polyols, polyisocyanates or silicones.

9. Device according to any one of claims 1 to 8, wherein the area ratio between the sensor area and the contact area of the sensor system is at most 1:4.

10. Device according to any one of claims 1 to 9, wherein the at least one sensor (101) comprises a transmitter unit.

11. Device according to any one of claims 1 to 10, wherein the viscoelastic medium (100) is enclosed with a polyurethane film and/or textiles for protection.

12. Device according to any one of claims 1 to 11, **characterized in that** the characteristic acoustic impedances of the viscoelastic medium (100) and of the adjacent material (116) at their boundary layer correspond to the inequality

$$\left|\frac{Z_{M3} - Z_{M2}}{Z_{M3} + Z_{M2}}\right| > 0,85$$

with

$Z_{M2}$ = characteristic acoustic impedances of the viscoelastic medium (100) and
$Z_{M3}$ = characteristic acoustic impedances of the adjacent material (116) are set.

13. Method for producing a device for detecting mechanical vibrations of human or animal tissues, in which at least one sensor (101) is embedded in a viscoelastic medium (100), the characteristic acoustic impedance of the viscoelastic medium being adapted to human or animal bodies, and the viscoelastic medium (100) having a characteristic acoustic impedance between 1400 and 2000 kNs/m$^3$, **characterized in that** a portion of the surface of the viscoelastic medium (100) is covered with an adjacent material (116) such that the adjacent material (116) leaves only one side of the viscoelastic medium (100) exposed for contact with the human or animal tissue (111), wherein there is no characteristic acoustic impedance between the underside of the medium and the adjacent material, so that a majority of the vibrations are reflected at the interface between the adjacent material and the medium.

14. The method according to claim 13, wherein the sensor (101) is embedded in the viscoelastic medium (100) in a free-floating manner.

**Revendications**

1. Dispositif pour détecter des vibrations mécaniques de tissus humains ou animaux (111), qui comprend au moins un capteur (101) qui est noyé dans un milieu viscoélastique (100), le capteur (101) et le milieu viscoélastique (100) formant un ensemble de capteurs (100, 101) et le milieu viscoélastique (100) forme une surface pour le contact avec le tissu humain ou animal (111), dans lequel l'impédance caractéristique acoustique du milieu viscoélastique est adaptée aux corps humains ou animaux, et dans lequel le milieu viscoélastique (100) présente une impédance caractéristique acoustique comprise entre 1400 et 2000 kNs/m$^3$, **caractérisé en ce qu'**une partie de la surface du milieu viscoélastique (100) est recouverte d'un matériau adjacent (116), le matériau adjacent (116) ne laissant qu'un seul côté du milieu viscoélastique (100) libre pour le contact avec le tissu humain ou animal (111), dans lequel il n'y a pas d'impédance caractéristique acoustique entre la face inférieure du milieu et le matériau adjacent, de sorte qu'une grande partie des vibrations est réfléchie à l'interface entre le matériau adjacent et le milieu .

2. Dispositif selon la revendication 1, dans lequel le capteur (101) est un capteur de pression.

3. Dispositif selon l'une des revendications 1 ou 2, dans lequel le capteur (101) est plan et sensible au signal des deux côtés.

4. Dispositif selon l'une des revendications 1 à 3, dans lequel le capteur (101) est noyé dans le milieu viscoélastique (100) de manière à flotter librement.

5. Dispositif selon l'une des revendications 1 à 4, dans lequel au moins un côté de la technique sensorielle (100, 101) est conçu de telle sorte qu'un contact à plat avec le tissu humain ou animal peut être établi avec ce côté.

6. Dispositif selon l'une des revendications 1 à 5, dans lequel le milieu viscoélastique (100) est constitué d'un matériau homogène.

7. Dispositif selon l'une des revendications 1 à 6, dans lequel le milieu viscoélastique (100) présente une densité comprise entre 900 et 1100 kg/m$^3$ et une dureté comprise entre 15 shore A et 40 shore A.

8. Dispositif selon l'une des revendications 1 à 7, dans lequel le milieu viscoélastique (100) est un composant choisi dans les groupes des carbamates, des polyols, des polyisocyanates ou des silicones.

9. Dispositif selon l'une des revendications 1 à 8, dans lequel le rapport de surface entre la surface du capteur et la surface de contact du capteur est au maximum de 1:4.

10. Dispositif selon l'une des revendications 1 à 9, dans lequel ledit au moins un capteur (101) comprend une unité d'émission.

11. Dispositif selon l'une des revendications 1 à 10, dans lequel le milieu viscoélastique (100) est entouré d'un film de polyuréthane et/ou de textiles pour le protéger.

12. Dispositif selon l'une des revendications 1 à 11, **caractérisé en ce que** les impédances caractéristiques acoustiques du milieu viscoélastique (100) et du matériau adjacent (116) au niveau de leur couche limite correspondent à l'inégalité

$$\left| \frac{Z_{M3} - Z_{M2}}{Z_{M3} + Z_{M2}} \right| > 0,85$$

avec

$Z_{M2}$ = impédances caractéristiques acoustiques du milieu viscoélastique (100)
$Z_{M3}$ = impédances caractéristiques acoustiques du matériau adjacent (116) sont réglés.

13. Procédé de fabrication d'un dispositif de détection de vibrations mécaniques de tissus humains ou animaux, dans lequel au moins un capteur (101) est noyé dans un milieu viscoélastique (100), l'impédance caractéristique acoustique du milieu viscoélastique étant adaptée aux corps humains ou animaux, et le milieu viscoélastique (100) présentant une impédance caractéristique acoustique comprise entre 1400 et 2000 kNs/m³, **caractérisé, en ce qu'**une partie de la surface du milieu viscoélastique (100) est recouverte d'un matériau adjacent (116) de telle sorte que le matériau adjacent (116) ne laisse qu'un côté du milieu viscoélastique (100) libre pour le contact avec le tissu humain ou animal (111), dans lequel il n'y a pas d'impédance caractéristique acoustique entre la face inférieure du milieu et le matériau adjacent, de sorte qu'une grande partie des vibrations est réfléchie à l'interface entre le matériau adjacent et le milieu.

14. Procédé selon la revendication 13, dans lequel le capteur (101) est noyé dans le milieu viscoélastique (100) de manière à flotter librement.

**FIG. 1A**

**FIG. 1B**

**FIG. 1C**

FIG. 2A

FIG. 2B

FIG. 2C

FIG. 2D

FIG. 2E

FIG. 2F

FIG. 2G

FIG. 3A

FIG. 3B

Rohsignal

103

| 106 |
|---|

| 107 |
|---|

| 108 |
|---|

| 109 |
|---|

| 110 |
|---|

**FIG. 4**

**FIG. 5**

**IN DER BESCHREIBUNG AUFGEFÜHRTE DOKUMENTE**

**In der Beschreibung aufgeführte Patentdokumente**

- WO 2017134681 A2
- WO 2007061619 A1
- DE 102013110900 A1
- WO 2016053398 A1
- WO 2018009165 A1
- US 2015230747 A1
- EP 1125975 B1
- WO 2007061619 A

**In der Beschreibung aufgeführte Nicht-Patentliteratur**

- **CHUO ; KAMINSKA.** *IEEE Transactions on Biomedical Circuits and Systems,* August 2009, vol. 3 (4